# EUROPEAN PATENT SPECIFICATION

(11) **EP 0 813 889 B1**
(45) Date of publication and mention of the grant of the patent: **27.05.2009**
(21) Application number: 97304267.4
(22) Date of filing: 18.06.1997
(51) Int. Cl.: A61N 1/368, A61N 1/39

(54) **System for cardiac stimulus steering**
System zur Herstimulussteuerung
Système de pilotage d'une stimulation cardiaque

(30) Priority: 18.06.1996 US 20421 P; 30.10.1996 US 755797
(43) Date of publication of application: 29.12.1997
(73) Proprietor: MEDTRONIC, INC., Minneapolis MN 55432-5604 (US)
(72) Inventor: Thompson, David L., Fridley, Minnesota 55432 (US); King, Gary W., Fridley, Minnesota 55432 (US); Hrdlicka, Gregory A., Plymouth, Minnesota 55447 (US)
(74) Representative: Hughes, Andrea Michelle

(56) References cited:
- EP-A- 0 538 990
- US-A- 5 174 289
- US-A- 5 324 327
- US-A- 5 344 430
- US-A- 5 441 518
- US-A- 5 447 519

## Description

The present invention relates to the field of cardiac stimulation systems, and more particularly to cardiac pacing and defibrillating systems having the capability to steer pacing or defibrillation pulses from a given electrode arrangement to different cardiac target sites to obtain enhanced efficiency and flexibility.

It has long been understood that efficient and reliable delivery of pacing and defibrillation pulses to a desired site is of great importance. Thus, even with the significant improvements that have been made in areas such as high efficiency batteries, low power consumption chip circuitry and programmability for optimizing pacing threshold, there remains a need to improve the delivery of the generated pulses to the cardiac tissue. If energy from a lead to a cardiac site is delivered inefficiently, the result is a greater effective threshold for capture, increased power drain and consequent decrease in pacemaker lifetime. Where failure to capture occurs, a desired pacing function is simply not performed. Backup circuitry and software can be implemented to detect loss of capture and to respond by delivering pacing pulses of greater energy. This approach, however, does not overcome the chronic performance problems so resulting, including the need to reprogram the pacemaker and accompanying patient discomfort and annoyance.

In conventional pacemaker implants, and especially in single chamber pacemaker implants, a suitable chronic threshold may be obtained by carefully anchoring the distal tip of the lead with respect to the patient's heart wall by using a tined lead or other fixation means, and then reprogramming the pacemaker soon thereafter to obtain a satisfactory chronic threshold. In such cases where only one site is being stimulated, and the portion of the lead carrying the electrode (or electrodes) can be anchored close to the site, the problem is minimal. In a dual chamber pacemaker system a second lead may be introduced into the atrium and also anchored. However, this complicates and lengthens the implant procedure and results in two thresholds having to be programmed. One answer to this problem was provided by the VDD pacemaker system with a single lead, the lead having a conventional electrode arrangement (either unipolar or bipolar) at its distal end, and a pair of "floating" electrodes positioned in the atrium. The floating atrial electrodes are not fixed to the atrial wall and consequently cannot provide the performance equivalent to fixed electrodes. As a result, such floating electrodes typically sense atrial signals but cannot deliver effective pacing pulses. Although some attempts have been made to design floating electrodes and pacing pulse waveforms to achieve atrial pacing, and thus provide a DDD system with a single lead, those attempts have not been very successful due to the separation of the electrodes from the intended atrial pacing site. In short, whenever electrodes are not well anchored at the pacing site the problems of achieving and maintaining an efficient pacing threshold are magnified.

The advent of certain newer types of pacing systems has further magnified the problem of achieving efficient pacing where the pacing electrode or electrodes cannot be affixed directly to the desired pacing site. One such system is multisite or biatrial pacing, where sensing or stimulation of the right and left atria improves the cardiac output of patients with inappropriate intra-atrial delays. This concept is described in U.S. Pat. No. 5,414,161. As disclosed in the 161 patent, right atrial sensing and pacing is accomplished using commonly available endocardial active fixation leads implanted in accordance with conventional right-sided DDD pacing practice. Left atrial sensing and pacing, however, is typically accomplished by placing either a tined endocardial lead or a lead like the Medtronic Model No. 2188 lead in proximity to the left atrium via the coronary sinus. It is commonly known that coronary sinus leads are difficult to place. That is, it is often difficult to determine an initial location in the coronary sinus where an adequate threshold may be obtained. Difficulties at implant resulted in lengthened implant procedures, patient discomfort, increased costs and higher thresholds, all of which reduce the longevity of the implanted device. Additionally, it is well known that coronary sinus electrodes often move or shift after implantation, resulting in chronic threshold "shift". This often results in loss of capture and requires the patient's pacemaker to be reprogrammed to higher pulse amplitudes or widths. This also results in a reduction in the longevity of the implanted pacemaker. Reprogramming the device often results in an inability to capture or the provision of an adequate safety margin. The physician must then reposition the lead during an additional surgical procedure, thereby increasing costs and risks of infection.

Another relatively unused but potentially advantageous pacing system is that of DDD pacing with only a single lead, where pacing and sensing are accomplished in both atrial and ventricular chambers with one lead carrying one or more electrodes. U.S. Patent No. 5,265,601 describes such a system for pacing the atrium and the ventricle from a single electrode positioned in the coronary sinus or deep cardiac vein. In such a system the stimulus amplitude is varied so that the atrial pacing pulse has a relatively low amplitude and the ventricular pacing pulse has a relatively high amplitude. The respective pulses are timed in accordance with the pacemaker's tracking of the cardiac cycle. While the foregoing concept provides acceptable results under some conditions, premature atrial contractions (PACs), premature ventricular contractions (PVCs), 2:1 block, oversensing of far-field R waves and the like can cause such systems to become confused, resulting in disrupted timing and loss of function. Additionally, the signal output in such a system is omnidirectional in respect of the electrode configuration. This results in threshold variation and low capture efficiency. Furthermore, due to the omnidirectional nature of the signal, either the V or A pacing pulse may cause unintended stimulation of the opposing chamber if it is stimulated outside the refractory period. Finally, such a system limits the maximum AV delay to a value less than the refractory period of the atrial myocardial tissue, because otherwise the V pace pulse would stimulate the atrium.

The above examples are not exhaustive of examples where a need exists to deliver stimulus pulse to desired cardiac locations in a better way. As discussed in more detail below, the ability to deliver stimulus pulses to desired locations with improved site resolution is desired in defibrillation systems, and in treating cardiac arrhythmias such as atrial tachycardia. What is needed is a system that provides the maximum flexibility and control without employing multiple leads having anchored electrodes at each specific site of interest.

An approach that has been taken in the area of spinal cord stimulation is disclosed in U.S. Patent No. 5,501,703. The '703 patent discloses a multichannel apparatus for epidural spinal cord stimulation. The invention is based on the observation that a stimulation induced paresthesia pattern may be focused and changed by using multiple electrodes and changing the parameters of the delivered stimulus pulses. The superposition of potential electric fields generated by simultaneous stimulation provided by multiple pulse generators and corresponding electrodes is described as resulting in a significant change in the size and shape of the stimulated spinal cord area. A lead is described as being implanted a few millimeters from the spinal cord with an electrode array disposed in the epidural space. Parametric changes of the delivered stimulus pulses provide some control over which nerve fibers adjacent to the electrode array are excited, and which are not. While this technique has been found successful in this limited area of spinal cord stimulation, no similar application has been developed for pacing or cardiac stimulation. Document EP-A-0 538 990 discloses the most relevant prior art.

What is desired for cardiac applications is a system to effectively "steer" cardiac stimulus pulses so that they efficiently capture or defibrillate a selected heart chamber, or otherwise control a cardiac arrhythmia. Such a system must take into account the fact that in cardiac applications the chambers are separate and are not similar to nerves aligned along a single spinal cord. Rather, cardiac muscle tissue in one or more separate chambers must be stimulated without having an array of electrodes positioned substantially adjacent to the various stimulus target locations.

According to the present invention, there is provided a pacing system as defined in claim 1.

According to one embodiment the invention may also comprise a pacing lead connected to said pulse generator means to receive said first and second pulses, said lead having a distal end portion adapted for positioning proximate to or in the patient's heart, said distal end portion having first and second active electrodes and a common electrode, and connecting means for connecting said first pacing pulses between said first electrode and said common electrode and for connecting said second pacing pulses between said second electrode and said common electrode; and
control means for controlling said pulse generator means to generate respective steered ventricular and atrial pacing pulses, each of which steered pulses is a combination of a said first and a said second pulse, and
steering means for controlling said first and second pulse means so as to select a first set of values of magnitude, polarity and phase for each of said first and second pulses when said pulse generator generates a ventricular pacing pulse, and a second set of values of magnitude, polarity and phase for each of said first and second pulses when said pulse generator generates an atrial pacing pulse.

According to one embodiment, the invention includes:
switch matrix means for connecting selected ones of said generator outputs to respective selected ones of said electrodes, and selected ones of said electrodes to selected ones of said sense amplifiers, said matrix connecting composite stimulus pulses consisting of plural pulse components to selected ones of said positions; and
pulse parameter and timing control means for controlling the connections made by said switch matrix means, whereby selected cardiac sites receive steered composite stimulus pulses and said composite stimulus pulses are timed as a function of sensed patient natural heartbeat signals.

The present invention provides system embodiments for pacing or otherwise stimulating one or more cardiac sites from a proximately positioned set of electrodes, where the individual pulse parameters of a plurality of pulse components are controlled in such a manner that the outputs are combined into composite stimulus pulses. The present invention permits efficient steering of each such composite pulse to a selected site and adjustment of steering parameters to adapt to threshold changes. By varying the field of stimulation over a range of cardiac areas, the present invention allows a quicker implant procedure because the lead may be positioned in a general area such as the left atrium or the coronary sinus. Later an optimum threshold for stimulating in one or more areas may be selected and programmed through setting the various pulse parameters corresponding to each pacing or defibrillation pulse component of each composite stimulus pulse.

In a general system configuration the present invention provides for at least one lead having three or more electrodes disposed thereon. During implantation the electrodes are positioned in a selected area within or proximate to the heart, and the composite stimulus pulses are targeted or steered to one or more selected cardiac sites. In a simple arrangement of the present invention, three electrodes are employed: two active electrodes and one common electrode (or "ground") deliver two pulses, the two pulses being components of a composite pulse programmed to generate an electric stimulation steered toward a selected site. The pulse components are programmed in respect of output pulse parameters that include magnitude (either amplitude or duration), polarity, and phase (timing). Each pulse is delivered between one of the active electrodes and the common electrode. In a more complex arrangement, the lead has a number of electrodes (n) greater than three, the electrodes being controllably connected through a switch matrix to be either active or common. This arrangement provides steering control through both pulse parameter control and electrode selection. The control may also incorporate switching delivery of stimulus pulses and sensing to either a bipolar or unipolar arrangement for further optimization of pulse capture and accurate sensing. In a further embodiment, spaced apart electrodes compare the relative timing of sensed signals and verify the origin of the sensed signal as being either in the ventricle or in the atrium.

In a first specific embodiment of the present invention, a system of left atrial pacing is provided, where a lead having an array of two active electrodes and one common electrode is positioned in proximity to the left atrium in the coronary sinus. Composite pacing pulses are generated by producing two pulses from two separate generators, where each pulse is controlled at least in respect of amplitude and is delivered between an active electrode and a common electrode. Pulse parameters are preferably controlled to steer the area of tissue stimulation so that it sweeps from right, to centre and to the left. Once output pulse parameters are optimized by standard threshold determination techniques, the values are stored in memory locations in the pacemaker. Optimizing output pulse stimulus parameters such as independently programmed pulse amplitude, pulse width, and degree of overlap (or phase) between pulses permits recapture and threshold optimization in the event of lead dislodgment. Thus, an additional surgical procedure for repositioning a migrated or dislodged lead becomes unnecessary.

A second embodiment of the present invention provides for DDD pacing from a single lead, the lead having at least two active electrodes and at least one common electrode positioned in the coronary sinus or deep cardiac vein. A different two pulse component composite stimulus is generated for each of the ventricular and atrial pulses, respectively. For the ventricular stimulus, a larger composite amplitude is employed and pulse is steered toward the ventricle by appropriate adjustment of the parameters of the pulse components delivered across each active electrode with respect to the common electrode. For the atrial stimulus, a smaller composite amplitude is employed and pulse parameters are varied to steer the resulting field toward the atrium. Pulses may be timed in accordance with the cardiac cycle, taking into account sensed signals from each chamber. The lead preferably has one or more additional sensing electrodes to enable discrimination of the direction from which heart signals emanate such that any ambiguities in respect of unusual signals such as PACs and PVCs may be resolved. In this embodiment of the present invention and others, the automatic threshold determination feature is preferably protected so that threshold variations may be used to automatically adjust pulse steering parameters for optimum performance.

In yet another embodiment of the present invention a single pass lead is used for DDD mode pacing, where the lead has one or two electrodes for pacing and sensing in the ventricle, and an array of floating electrodes positioned in the atrium for pacing and sensing in the atrium. The atrial array preferably has three electrodes: two active electrodes and one common or indifferent electrode. Different pulses are delivered across each active/common pair, either simultaneously or with very little phase difference. Pulse parameters are adjusted as required to permit optimal steering of pacing pulses for enhanced stimulation.

In addition to the above embodiments of the present invention the system of the present invention may be utilized in other embodiments directed to atrial defibrillation, atrial arrhythmia prevention, ventricular defibrillation, and site specific anti-tachycardia therapy. The system of the present invention may also employ specific lead and pulse output subsystems.

Preferred embodiments will now be described by way of example only, with reference to the accompanying drawings.
Figures 1a to id illustrate a patient's heart and show alternative embodiments of the lead employed in the system of the present invention for steering stimulus pulses to different cardiac sites;
Figure 2 shows a block diagram of the primary system components of the present invention for steering pulses from electrodes positioned within or proximate to the patient's heart to one or more cardiac sites;
Figure 3A shows a block diagram of a pacing system of the present invention that provides stimulus pulses steered to a patient's left atrium and other cardiac sites as part of a multi-chamber pacing system;
Figure 3B shows a block diagram of the present invention, where details of the output generator portion of the system of Figure 3A are shown;
Figure 3C shows a timing diagram that illustrates variations in the pulse amplitude of the components of the composite stimulus pulse of the system of Figure 3A, where the variations causes teering of the composite pulse to different atrial locations;
Figure 3D shows a block diagram of a pacing system of the present invention that provides stimulus pulses to various portions of a patient's heart, including the left or right atria and the right ventricle.
Figure 3E shows a block diagram of the present invention, where details of the output generator of teh system of Figure 3D are shown.
Figure 4A shows a distal end portion of a lead of the present invention positioned proximate the heart, where the lead has plural electrodes for delivery of respective pacing pulses steered to different cardiac sites;
Figure 4B shows a timing diagram illustrating the delivery of one composite steered pulse per cardiac cycle;
Figure 4C shows a timing diagram illustrating the delivery of two composite steered pulses per cardiac cycle;
Figure 4D shows a timing diagram illustrating the delivery of three composite steered pulses per cardiac cycle;
Figure 4E shows a block diagram of a system modification for detecting the origin of a sensed signal and to provide enhanced discrimination of sensed atrial and ventricular signals;
Figure 5 shows a flow diagram of a routine of the present invention for carrying out a closed loop adjustment of steering parameters;
Figure 6A shows a sketch of a distal portion of a typical prior art single pass lead used for delivering omnidirectional bi-phasic stimulus pulses from a floating atrial electrode;
Figure 6B shows a flow diagram of a routine for the present invention for providing closed loop adjustment of steering parameters for use with the lead of Figure 6A;
Figure 7A shows a perspective diagram of a lead of the present invention having electrodes positioned to apply a steered atrial defibrillation or cardioversion stimulus to the atrium;
Figure 7B shows a perspective view of a pair of leads of the present invention having electrodes positioned to apply a steered ventricular defibrillation or cardioversion stimulus to the ventricle;
Figure 7C shows a block diagram of the primary components of a system for the present invention for delivering a steered defibrillation or cardioversion stimulus to the electrodes of Figure 7A or 7B;
Figure 8 shows a flow diagram of the primary steps of the present invention in a routine for applying morphology specific cardiac therapies using steered electrical stimuli;
Figure 9A shows a alternative three electrode embodiment of the present invention for applying steered pulses to one or more cardiac targets;
Figure 9B shows an alternative five electrode embodiment of the present invention for applying steered pulses to one or more cardiac targets; and
Figure 9C shows an alternative electrode embodiment of the present invention or applying steered pulses to one or more cardiac targets, where a tubular endocardial lead is employed.

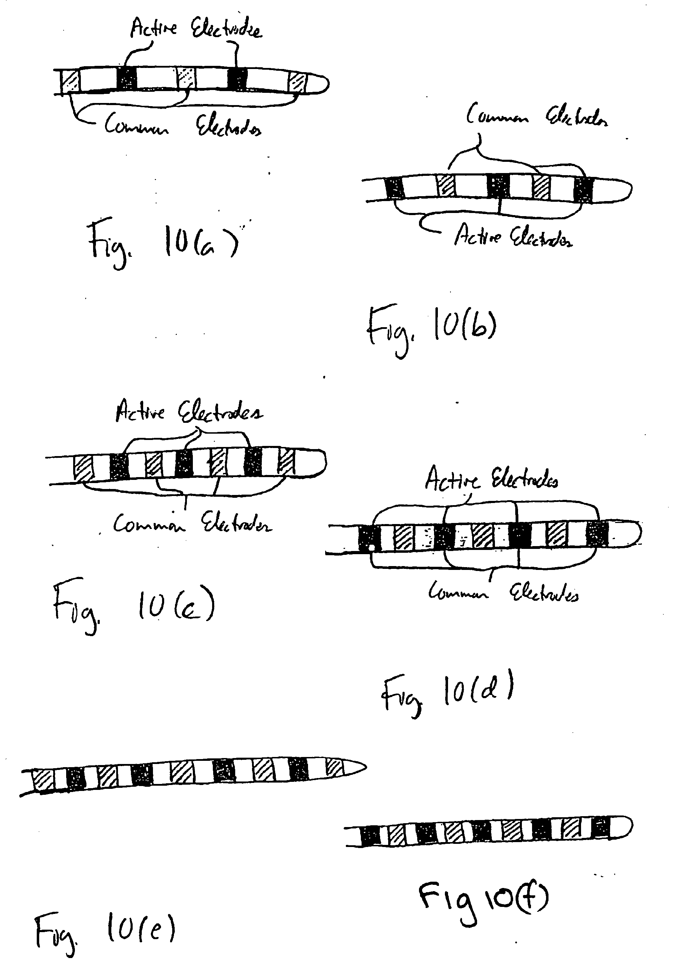

Figure 10A through 10F show different embodiments of multi-electrode leads falling within the scope of the present invention, where different numbers of active and common electrodes are arranged in various configurations respecting one another.

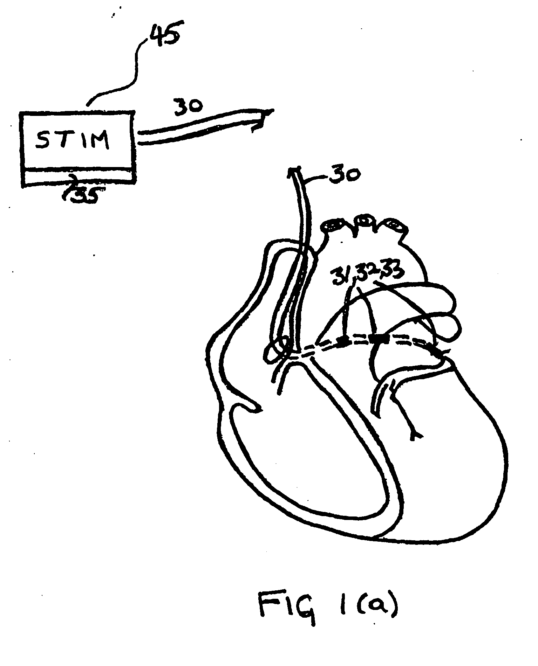

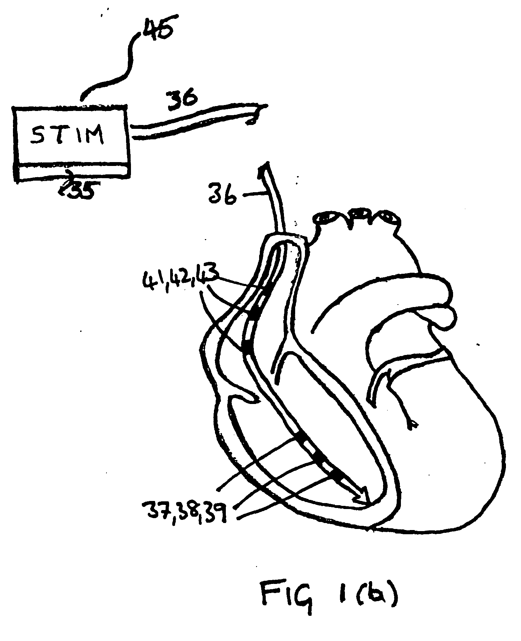

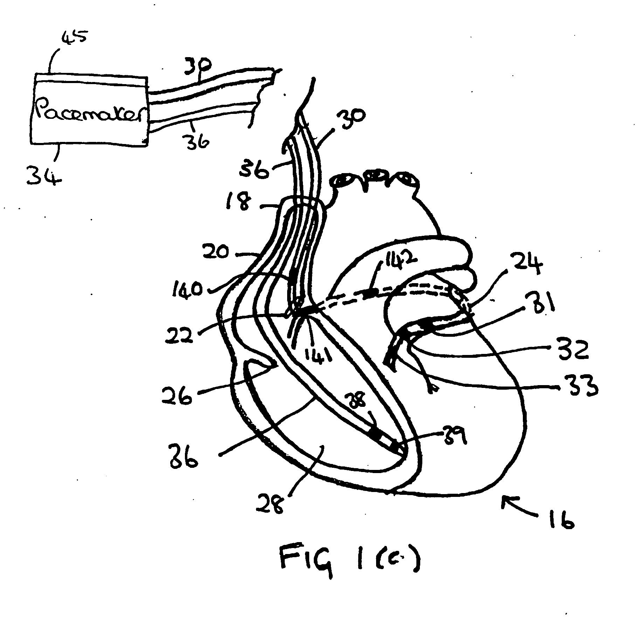

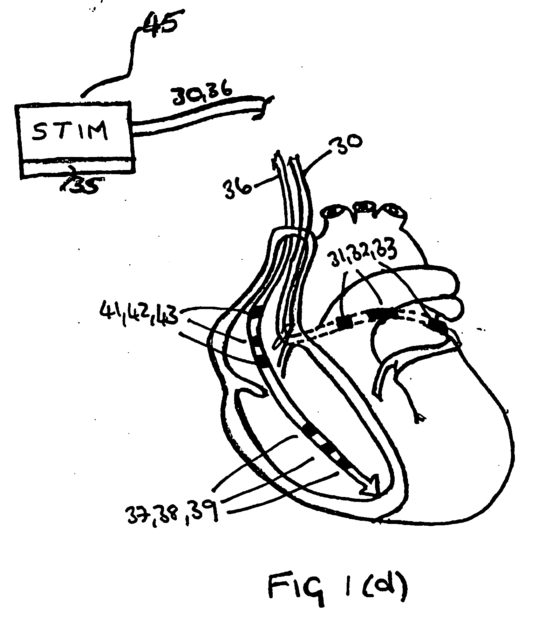

Figures 1a to 1d show a schematic diagram of a patient's heart with different leads for delivering composite stimulus pulses steered to specific sites in accordance with the present invention. Figure 1 is illustrative, and is not intended to suggest all embodiments of the present invention. In Figure 1(a) lead 30 is positioned with its distal end portion in the patient's coronary sinus, and has three electrodes 31,32,33, where electrode 32 is preferably a common electrode. In addition, stimulator case 35 may be used as a common electrode. Lead 30 is connected to stimulator 45 which is a pacemaker, a defibrillator, or a combined pacemaker-cardioverter-defibrillator stimulator. Lead 30 has conductors connecting pulse generator outputs from stimulator 45 to corresponding electrode pairs 31,32 and 33,32 for delivering composite pulses. Individual pulse parameters are chosen to steer the composite pulse to a specific site. For example, pulses may be steered to the right or left atrium from the location shown in Figure 1 or to both the atrium and the ventricle. A second lead 36 is also shown in Figure 1b. Lead 36 is usually used alone, but may be used in conjunction with lead 30. Lead 36 is likewise connected to stimulator 45 and may have either or both electrode sets 37,38,39 and 41,42,43. Ventricular electrodes 37,38,39 are preferably connected to receive pulse component pulses across pairs 37,38 and 39,38 or may be connected between any one of such electrodes and case 35. Atrial electrodes 41,42,43 are preferably connected to receive pulse component pulses across pairs 41,42 and 43,42 or between any one of such electrodes and case 35. In any selected electrode configuration appropriate control of the pulse component parameters is utilized to steer the resulting composite pulse to the desired location. For example, floating atrium electrodes 41,42,43 may be used for atrial pacing in a DDD mode with single pass lead 36. In any particular electrode configuration, selected or additional electrodes may sense cardiac signals. Although electrode sets of two active electrodes and one common electrode (e.g., two anodes and one cathode) have been illustrated, the electrode sets may comprise a greater number of electrodes (n) and may be switchable in conjunction with the pacemaker or stimulator case as a common electrode. For intra-cardiac pacing applications, electrode surface areas are preferably approximately 34-50 mm^{2.} Separation between each active electrode and a common electrode preferably exceeds about 7 mm. For electrode placement outside a cardiac chamber (such as in the coronary sinus), the separation between each neutral electrode and a common electrode preferably exceeds about 1 cm. The corresponding distance between two active electrodes preferably exceeds about 1.4 cm, and most preferably exceeds about 2 cm. In defibrillation and other cardioversion applications, electrode surface areas and interelectrode spacings are greater.

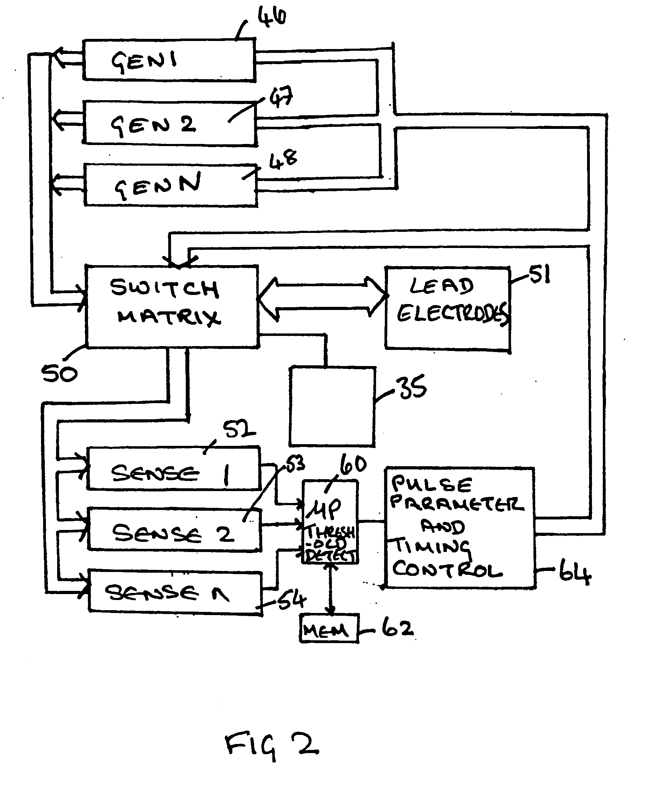

Figure 2 shows a block diagram of the primary components of stimulator 45 of the present invention. Generators 1,2, . . . n are shown at 46,47,48 and are generators for producing pulse components. The generators are enabled or not, and are controlled in respect of pulse parameters and timing by pulse parameter and timing control block 64. The selected generator outputs are connected through switch matrix 50 (also controlled by block 64) so that selected pulse components are connected to selected electrodes as shown at 51; one or more generator outputs may be connected between a selected electrode and stimulator ground 35. Sensed signals are routed through switch matrix 50 to sense circuits 52,53,54, the outputs of which are connected to microprocessor/threshold detect block 60. Block 60 performs all the usual and necessary logic and timing functions of a stimulator, and also detects when a delivered pulse has resulted in capture. Block 60 also determines threshold, and is connected to memory 62, where data relating to steering parameters are stored. Parameter data are generated internally as a function of the threshold detection or are received from an external programmer. As a result of the parameter data and timing determined at block 60, data for pulse parameter and timing control are coupled to block 64. A closed loop system for controlling the pulse parameters of the delivered stimulus pulses is thus provided.

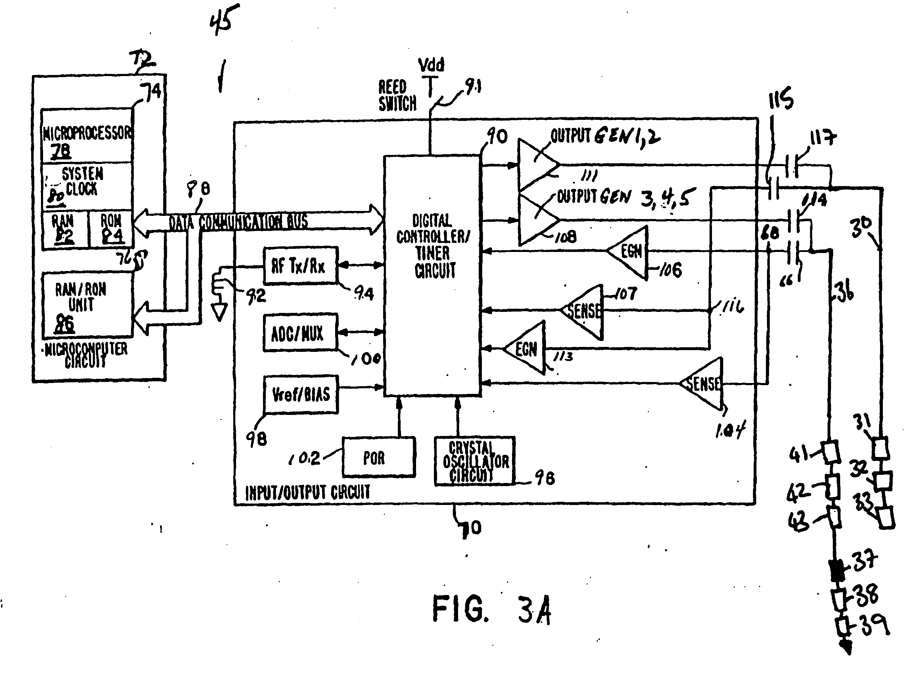

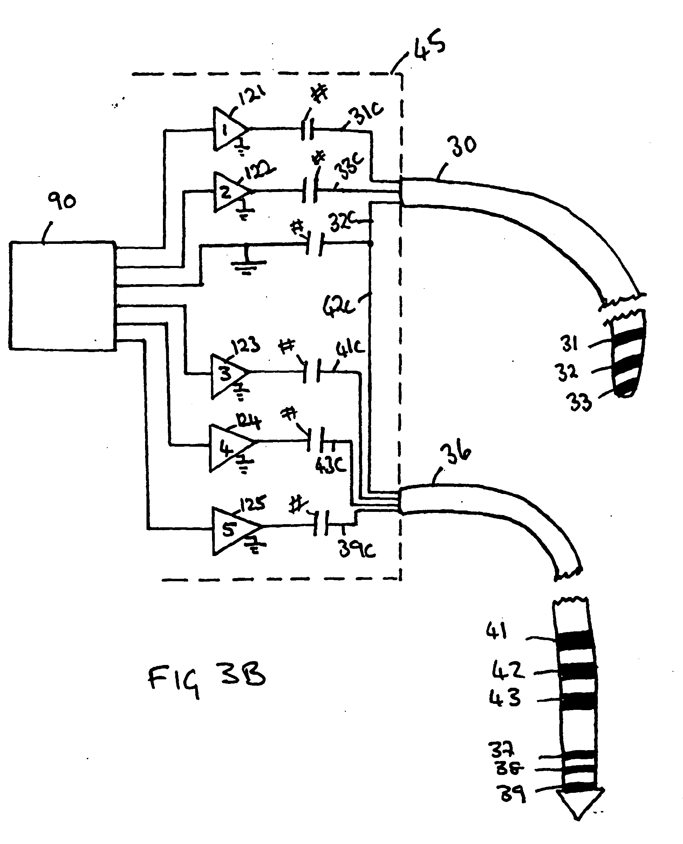

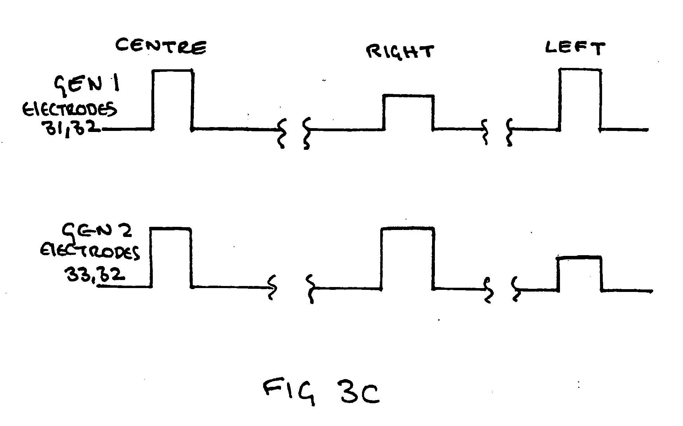

Figures 3A, 3B and 3C show a multichamber pacemaker having the steering feature of the present invention, where the left atrium may be paced with steered pulses. Figure 3A is modified in respect of Figure 2 in U.S. Patent No. 5,441,525. Although the present invention is described in accordance with a microprocessor-based architecture, it will be understood that it may be implemented using other technology such as digital logic-based, custom integrated (IC) architecture, or with any other combination of hardware and software familiar to those of ordinary skill in the art.

In Figure 3A pacemaker 45 is modified in respect of a conventional DDD pacemaker to provide multiple generators (GEN 1,2 and GEN 3,4,5) for generating a plurality of component composite steering pulses for pacing the left atrium, the right atrium or the right ventricle. Generators 1 and 2, shown jointly at 111, have their outputs coupled through at least one capacitor 117 to lead 30 for delivering pulse components between electrode pairs 31,32 (common) and 33,32. The parameters of each pulse component are adjusted to accurately steer the composite pulse so that the left atrium is captured. Generators 3 and 4 (shown at 108) likewise have their outputs connected to lead 36 for delivering steered pulses to the right atrium across electrode pairs 41,42 and 43,42. Generator 5 (also shown at 108) is connected to distal electrode 39 for unipolar pacing of the right ventricle, or between electrodes 39 and 38 for bipolar pacing.

Input/Output circuit 70 contains the input and output analog circuits and digital controlling and timing circuits necessary for the detection of electrical signals originating in the heart and sensed by sensors (not shown) connected to leads 30 and 36. Input/output circuit 70 further contains the circuitry required for the application of steered stimulating pulses to the heart under the control of software-implemented algorithms in Microcomputer Circuit 72.

Microcomputer Circuit 72 comprises an On-Board Circuit 74 and an Off-Board Circuit 76. On-Board-Circuit 74 includes microprocessor 78, system clock 80, and on-board RAM 82 and ROM 84. Off-Board Circuit 76 includes off-board RAM/ROM Unit 86. Microcomputer Circuit 72 is coupled by Data Communication Bus 88 to Digital Controller/Timer Circuit 90. Microcomputer Circuit 72 may be fabricated of custom IC devices and augmented by standard RAM/ROM components. It will be understood by those skilled in the art that the electrical components represented in Figure 3A are powered by an appropriate implantable-grade battery power source that is not shown explicitly..

Antenna 92 is connected to Input/Output Circuit 70 for uplink/downlink telemetry through radio frequency (RF) Transmitter/Receiver Circuit (RF TX/RX) 94. Telemetering both analog and digital data between antenna 92 and an external device such as an external programmer (not shown) is accomplished in a preferred embodiment by means of all data first being digitally encoded and then pulse position modulated on a damped RF carrier, substantially as described in U.S. Pat. No. 5,127,404 issued on July 7, 1992, and entitled "Telemetry Format for Implantable Medical Device,". Reed switch 91 is connected to Input/Output Circuit 70 to enable patient follow-up through telemetry and programming functions.

Crystal Oscillator Circuit 96 (typically a 32,768 Hz crystal-controlled oscillator) provides main timing clock signals to Digital Controller/Timer Circuit 90. Vref/Bias Circuit 98 generates a stable voltage reference and bias currents for the analog circuits of Input/Output Circuit 70. ADC/Multiplexer Circuit (ADC/MUX) 100 digitizes analog signals and voltages to provide telemetry and a replacement time-indicating signal or end-of-life function (EOL). Power-On-Reset Circuit (POR) 102 functions to initialize pacemaker 40 with programmed values during power-up, and reset the program values to default states upon the detection of a low battery condition, or in the presence of certain undesirable conditions such as unacceptably high electromagnetic interference (EMI).

The operating commands for controlling the timing of the pacemaker depicted in Figure 3A are coupled by bus 88 to Digital Controller/Timer Circuit 90, where digital timers set the overall escape interval of the pacemaker and the various refractory, blanking and other timing windows for controlling the operation of peripheral components in Input/Output Circuit 90.

Digital Controller/Timer Circuit 90 is coupled to sense amplifiers (SENSE) 104 and 107 and to electrogram (EGM) amplifiers 106 and 113 for receiving amplified and processed signals representative of the electrical activity of the patient's ventricle and atrium, respectively. Those signals are sensed by one or more electrodes 31, 32, 33, 37, 38, 39, 41, 42, 43, depending on which cardiac sites are being paced. Although only two sense amplifiers are shown in Figure 3A, a plurality of sense amplifiers and a switching matrix may also be employed, as, for example, shown in Figure 2 at 50 and 52,53,54. See also U.S. Patent No. 5,423,873, where a switching arrangement for switching pacemaker output and input terminals to different electrodes in a multi-electrode arrangement is disclosed. SENSE amplifiers 104 and 107 produce sense event signals for resetting the escape interval timer within Circuit 90. The electrogram signal provided by EGM amplifier 106 may be used when the implanted device is being interrogated by the external programmer/transceiver (not shown) to transmit by uplink telemetry a representation of the analog electrogram of the patient's electrical heart activity. See, for example, U.S. Pat. No. 4,556,063 to Thompson et al., entitled "Telemetry System for a Medical Device,".

The plurality of output pulse generators represented at 108 and 111 provide pacing stimuli to the patient's heart through output capacitors 114 and 117 and leads 30 and 36 in response to pacing trigger signals output by Digital Controller/Timer Circuit 90 each time an escape interval times out, an externally transmitted pacing command is received, or in response to other stored commands. As discussed above, each delivered pulse is, in fact, a composite of a plurality of component pulses, where each component pulse has a particular programmed or adjusted set of amplitude, duration (width) and phase parameters associated with it.

Figures 3B and 3C show an arrangement for generating and steering the pulses in greater detail. In Figure 3B, generators 1-5 are controlled by control circuit 90 in respect of timing and amplitude, duration and phase parameters. Generator 1 at 121 is coupled through capacitor C1 to lead conductor 31C connected to the output through lead 30 to electrode 31. The output of generator 2 at 122 is coupled through capacitor C2 on conductor 33C to electrode 33. The pacemaker ground or common is connected through capacitor CC on conductor 32C to electrode 32. Pulse parameter control circuit 90 determines the direction the composite pulse will have when it is delivered to the left atrium, as illustrated in Figure 3C. Thus, for a centrally directed pulse the amplitudes across pairs 31,32 and 33,32 are substantially equal. For a right-directed pulse the amplitude across electrodes 33,32 is greater than that across electrodes 31,32. For a left-directed pulse the relative amplitudes are switched so that the pulse originating in generator 1 and delivered across electrodes 31,32 has a greater amplitude than that delivered across electrodes 33,32. Output pulse parameters may be adjusted by standard threshold determination techniques and optimized parameters stored in memory.

Pulses delivered by lead 36 are generated by generators 123, 124 and 125 and coupled through capacitor C3 and conductor 41C, capacitor C4 and conductor 43C, and capacitor C5 and conductor 39C, respectively, to electrodes 41,43 and 39. While not shown specifically, pulses generated and delivered across electrode pairs 41,42 and 43,42 are likewise adjusted in amplitude, duration or both to optimize pacing of the right atrium from those floating electrodes. Since the distal end of lead 36 may be anchored in the apex of the right electrode, there is no need to generate composite steering pulses for delivery to electrodes 39,38. Of course, atrial and ventricular pace pulses are controlled in respect of the time sequence in which they are delivered (as in a conventional DDD pacemaker).

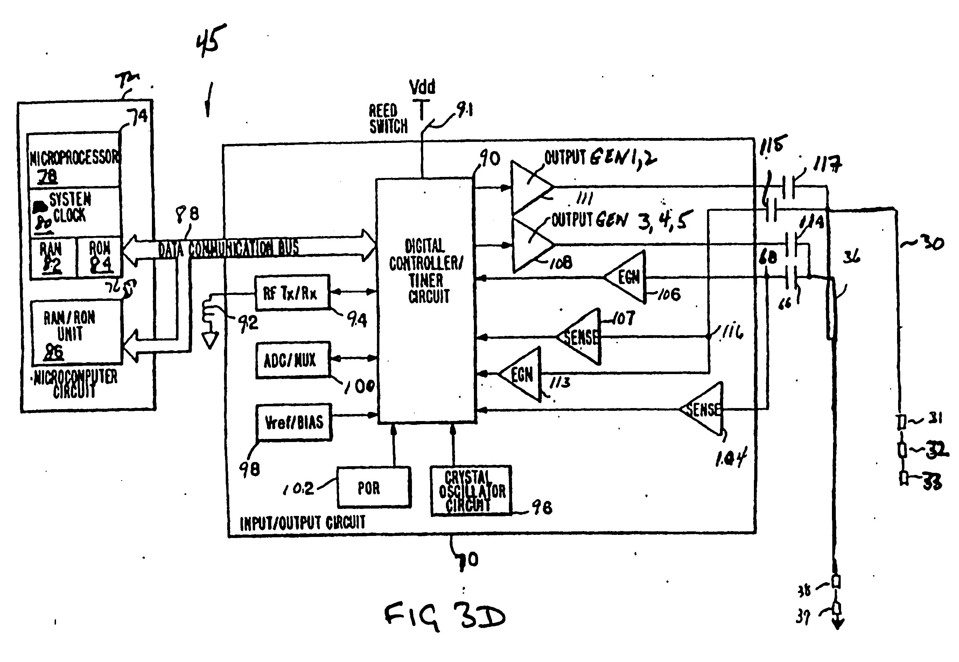

Figure 3D shows a block diagram of a pacing system of the present invention that provides stimulus pusles to various portions of a patient's heart, including the left or right atria and the right ventricle.

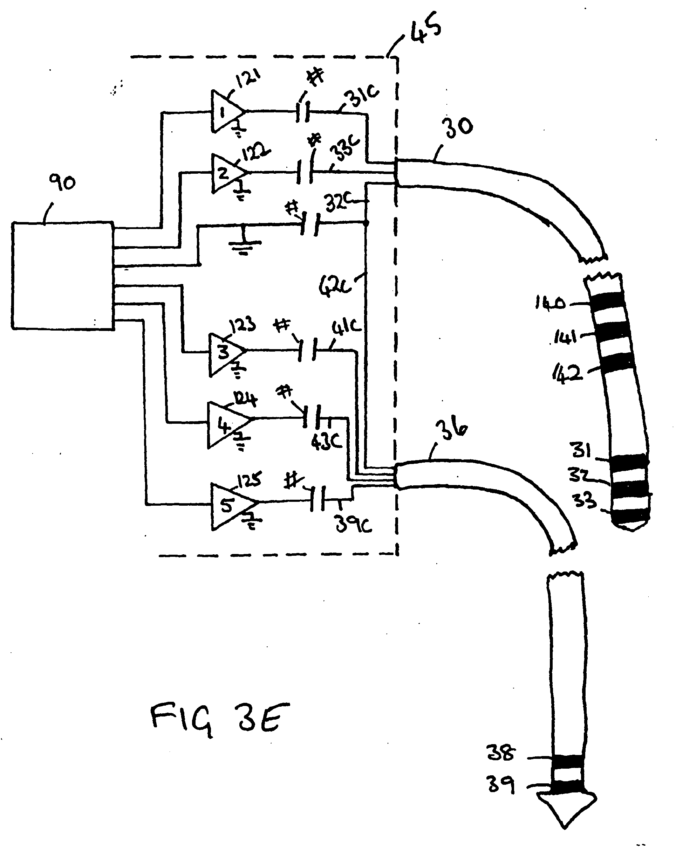

Figure 3E shows a block diagram of the present invention, where details of the output generator portion of the system of Figure 3D are shown.

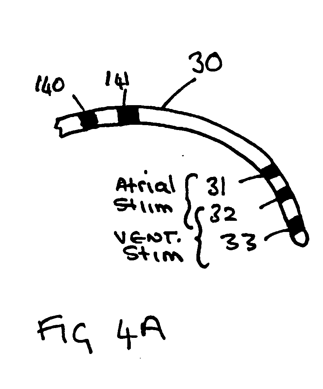

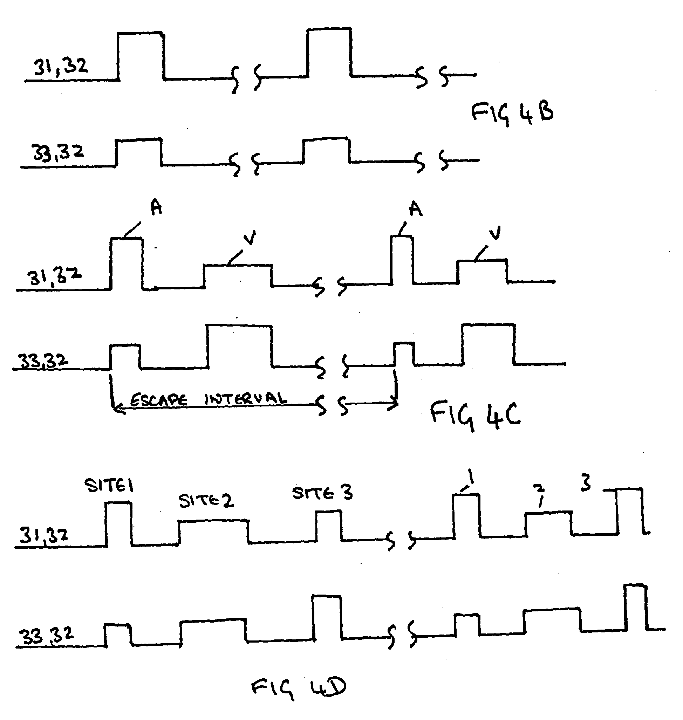

Figures 4A, 4B, 4C and 4D illustrate an arrangement and method for multi-site pacing from a single lead. We also refer now to the pacemaker shown in Figures 3A and 3B in respect of the generation of pulse signals and to U.S. Patent 5,265,601. The distal end portion of lead 30 is positioned in the coronary sinus or deep cardiac vein as illustrated in Figure 4A, and is connected to pacemaker 45. For pacing in both the atrium and the ventricle, electrode pair 31,32 delivers atrial pacing pulses, while pair 33,32 delivers ventricular pacing pulses. The individual pulse components are preferably generated by generators 121 and 122 (as shown in Figure 3B) and are controlled appropriately in respect of amplitude, width and phase.

Pacing of one, two or more sites may be accomplished as illustrated in Figures 4B, 4C and 4D. Figure 4B shows a single composite pulse being generated for each cycle, and may be employed to pace the left or right atrium. Steering is accomplished by adjusting the relative magnitudes of the component pace pulses. That is, the magnitude of the signal delivered across electrode pair 31,32 exceeds that delivered across electrode pair 33,32.

Figure 4C illustrates a pulse train for pacing two cardiac sites for each cycle and may be employed to pace the atrium and ventricle separately. The first pulse components have unequal amplitudes, phases or widths, resulting in steering of the electrical field in a first direction. The second pulse components have equal amplitudes, phases or widths, resulting in a straight-ahead field pattern. For combined atrial and ventricular pacing, composite atrial stimulating pulses may be of relatively low magnitude so that the ventricle is not stimulated. Likewise, the composite ventricular pulses may be of relatively high magnitude but may be delivered during the heart's natural refractory period so that only ventricular stimulation results. The atrial sense amplifier (or amplifiers) may be adjusted to have a lower threshold for sensing than the ventricular amplifier, so that lower-magnitude atrial signals exceed only the threshold of the atrial amplifiers, while higher-magnitude ventricular signals exceed the threshold of both amplifiers and may thus be recognized with appropriate logic. See, for example, U.S. Patent No. 5,265,601.

Figure 4D illustrates a waveform for stimulating three sites, where for each cardiac cycle three different composite pulses are generated and delivered across electrode pairs 31,32 and 33,32. A third composite pulse is delivered having a greater voltage across electrode pair 33,32 than across electrode pair 31,32 so that the first and third composite pulses are directed in different directions. Note also that steering may be aided by changing slightly the phase of the individual component pulses.

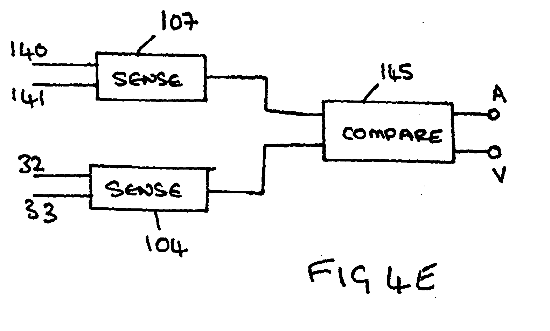

Figures 4A and 4E show an embodiment of the present invention where the capability of a single lead system to determine when a sensed signal has originated in the atrium and when it has originated in the ventricle is enhanced, thereby permitting better and more reliable pacemaker operation. Lead 30 has a pair of sense electrodes 140,141 positioned proximal to the distal electrodes so that when lead 30 is implanted the electrodes are positioned close to the atrium. Due to this relative positioning, a signal originating in the atrium is detected at electrode pair 140,141 before it is detected at electrode pair 32,33. Likewise, a signal originating in the ventricle is detected at electrode pair 32,33 before it reaches electrode pair 140,141. As shown in Figure 4E, the signals from the two electrode pairs are sensed at electrode pair 104,107 and time-compared at compare block 145. Such a comparison may be accomplished simply by detecting the leading edge of the incoming signal and setting a flip-flop to output signal A for a signal of atrial origin and to output signal V for a signal of ventricular origin. Alternatively, a Doppler detector and signal processor may determine the location from which a sensed signal has originated. Such a Doppler detector may be employed instead of or in addition to electrode pair 140,141 for sensing pressure waves resulting from a cardiac chamber contraction.

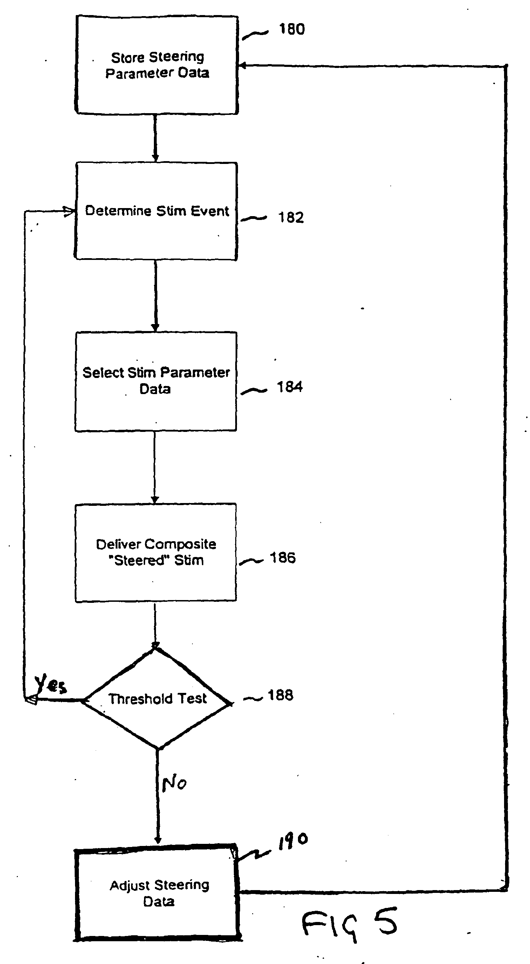

Figure 5 presents a simplified, generic flow diagram of the primary steps involved in practicing another embodiment of present invention. As illustrated at 180, steering data (or pulse component parameter data) are stored in the pacemaker. The data may be acquired initially by external programming or may be generated automatically by threshold testing.

In cardiac pacing applications, the amplitude of each pulse component is preferably varied in about 0.5 volt increments through a range of about 0.5 to about 7.5 volts; pulse widths are preferably varied in about 0.03 ms increments through a range of about 0.03 to about 1.5 ms; phase delays of one pulse relative to another are preferably varied in about 0.03 ms increments over a range of about -1.0 to about +1.0 ms.

Amplitude variations for defibrillation are much greater. Amplitude components may be varied in about 25 or about 50 volt increments over a range of about 25- about 750 volts.

At 182 the stimulus is to be delivered, (e.g., a ventricular or atrial pulse, a left atrial pulse, a defibrillation pulse, etc.). After the stimulus type is selected, at 184 appropriate parameter data are selected for the steered pulse. At 186 the plural component pulse is generated and delivered. At 188 a threshold test is performed to determine whether capture has occurred, and to compare the delivered pulse with the stored threshold pulse. At 190 steering parameters may be adjusted according to the results of the threshold test. If such an adjustment is made, one or more pulse parameters are adjusted at 192.

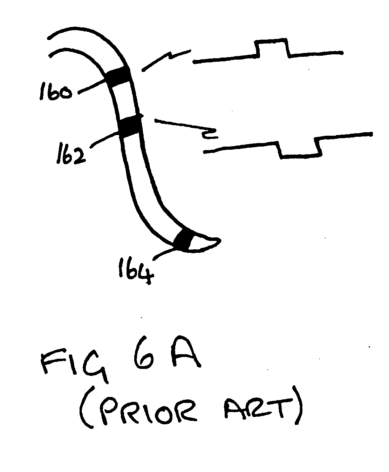

From Figures 3A and 3B, it can be seen that use of steering pulses in accordance with this invention can enable an improved variation of a VDD type pacemaker system which uses a single pass lead such as illustrated at 36. It is known to attempt to pace the atrium from a pair of floating electrodes in the atrium, which when done successfully provides an effective DDD pacing system with one floating lead. In such a prior art arrangement, as shown in Figure 6A, the single pass lead has two floating atrial electrodes and one or two electrodes 164 at or near the distal tip for pacing and sensing in the ventricle. It has been disclosed to utilize two opposite phase overlapping pulses, to provide a composite biphasic pulse. Each pulse is delivered between an electrode 160 or 162 and the pacemaker can, or alternatively 160 and 162 can each be a pair of electrodes. However, such a biphasic pulse is omnidirectional; while magnitude can be adjusted to try to achieve capture, as with any electrode arrangement, it is desirable to be able to steer the atrial pace pulses and to find the best direction for capturing the atrium. By using the pacemaker and lead 36 combination as described above, such a single pass DDD improvement of a VDD system can be accomplished. Stating it in the alternative, the use of the system of Figures 3A and 3B, but with only lead 36, provides such an improvement over the prior art VDD system.

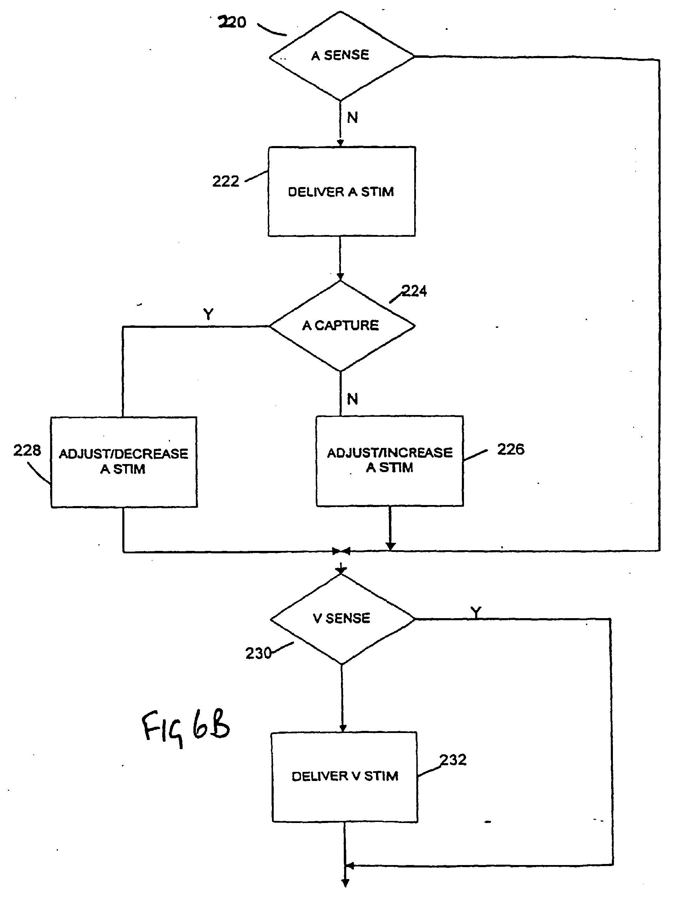

Figure 6B illustrates a simple flow diagram for executing the steps of a closed loop system for assuring capture for a DDD system with a single pass lead, and using steered pulses in accordance with this invention. At 220, it is determined whether there has been an atrial sense within the prescribed atrial escape interval. If yes, then no atrial stimulus is required, and the routine branches to 230 to look for a ventricular sense. But if no atrial signal is sensed, the routine goes to 222 and delivers an atrial stimulus steered for optimum capture. At 224, it is determined whether there has been capture. If yes, one or more pulse parameters can be changed to decrease the stimulus as shown at 228; if not, then at 226 the atrial stimulus parameters are changed to more effectively steer the pulse, or simply to increase the composite pulse level. At 230, the pacemaker waits to see if there has been a sensed ventricular signal before time-out of the ventricular escape interval. If yes, the routine exits; if no, a ventricular stimulus is delivered. Although not shown, the pacemaker can go through a capture detection routine and threshold adjustment for the ventricular pulse as well.

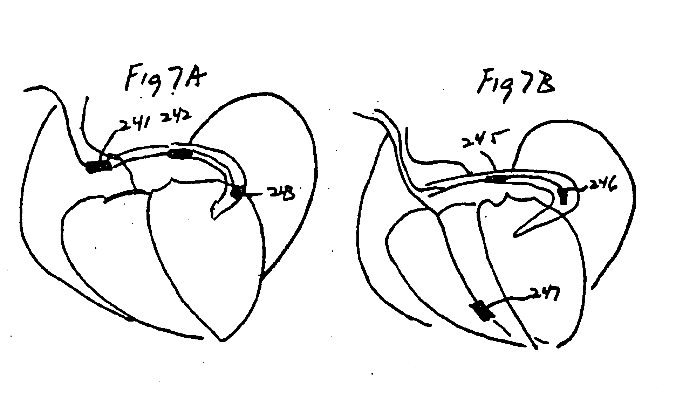

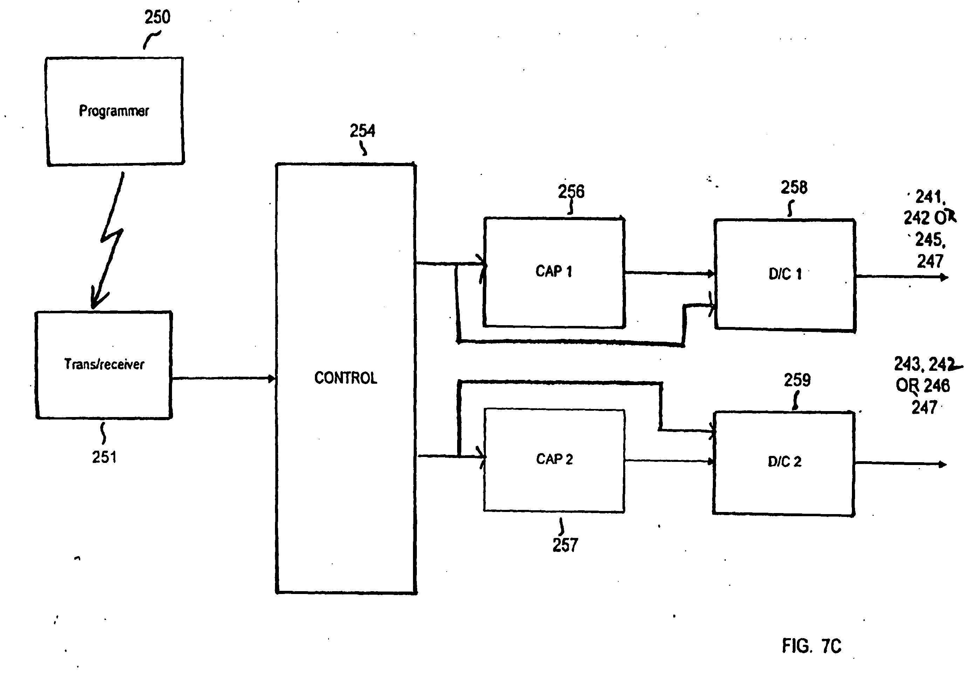

Figures 7A, 7B and 7C illustrates additional embodiments of the present invention for preventing defibrillation and arrhythmia. Atrial defibrillators are known and used in the art, and play an important role for patients prone to atrial defibrillation. See U.S. Patent No. 5,269,298, for an example of an atrial defibrillation system. As is known, a major drawback of current defibrillators is the pain associated with the shock that is applied, typically of the order of 2 joules. A lead typically used for atrial defibrillator applications is a coronary sinus lead, similar to lead 30 illustrated in Figure 1, but having electrodes adapted to delivering the larger defibrillation pulse or pulses. In this application also, lead dislodgment is a problem, since even a small movement of the electrode positions may result in a need to reposition the lead via surgical intervention or to reprogram the defibrillator to deliver a pulse having more energy. Either type of such intervention increases the patient's pain and anxiety for each shock therapy delivered. In an embodiment of the present invention, a lead having plural electrodes such as shown at 241,242,243 in Figure 7A is positioned at initial implant, and the threshold is optimized by adjusting the component pulse parameters for the respective pulses across electrode pairs 241,242 and 243,242. The stimulus can thus be optimized for optimum chronic delivery, including programming the stimulus to defibrillate the atrium only, thus improving over prior art systems where the stimulus shock must be closely timed to avoid affecting the ventricle. If the lead does migrate with time, the pulse parameters can be re-programmed, to regain an optimized composite stimulus shock. Referring to Figure 7C, there is presented a modification of a portion of the defibrillator circuit shown in the figure of referenced patent US 5,269,298, whereby the circuit is adapted for use with this invention. Here, a programmer 250 is shown, which communicates with transmitter/receiver 251, for reprogramming the pulse steering parameters. The parameter data is coupled to control 254, which controls the timing and parameter mix of the defibrillation pulses. As shown, a first capacitor 256 is charged, and discharged through circuit 258 to produce a first component across electrode pair 241,242; and a second capacitor 257 is charged, and discharged through circuit 259, to provide a second component across electrode pair 243,242. The control can, for example, control the charge on each capacitor, and thus the magnitude of the component pulse; and also the duration of the discharge.

Figures 7B and 7C illustrates a two lead system of the present invention for delivering defibrillation pulses to the ventricle and for steering those pulses to optimize shocking of the ventricle only. In this system, a first lead provides a pair of electrodes 245,246 positioned in the coronary sinus, and a common or indifferent electrode 247 in the ventricle (along with a standard RV pacing and sensing electrode 248). In this case, the steering pulses are applied across respective electrode pairs 245,247 and 246,247, to deliver optimized pulse components to the ventricle, adjusted as to their pulse parameters to accurately steer the pulse to the ventricle alone. Here again, the parameters can be adjusted by programming, in the event of lead migration after implant. Electrode pair 247,248 is used for standard right ventricular pacing and sensing, as is well known in the art.

The system in Figures 1 and 2 may also be modified to provide for optimized prevention of atrial arrhythmias. Reference is made to U.S. Patent No. 5,403,356, which describes a method of pacing the triangle of Koch and/or an area of prolonged effective refractory period elsewhere within the atrium to prevent atrial tachyarrhythmias. In that system, two leads are used, one to place an electrode pair at or in the triangle of Koch area, and one to place a pair of electrodes in the right atrial appendage. Although the electrode placement can be varied, there is always the problem of lead migration or chronic change in stimulus threshold, a problem which can be reduced by using the steering technique of this invention. The system of the referenced patent is modified in a manner similar to that shown in Figures 7A-7C, or in Figure 3B, i.e. by adding an additional output stage and providing a three electrode configuration on the first lead.

U.S. Patent No. 5,447,519, describes a method and apparatus for the detection of monomorphic and polymorphic arrhythmias and the selection of a therapy based upon that detection. In another embodiment of this invention, each such selected therapy can utilize the steering technique that has been described, i.e.,, steering capability is added to the morphology selected specific therapy. As described in the '519 patent, morphology specific therapies are selected at implant and stored along with the appropriate waveform used to break up a specific tachyrhythmia. In accordance with this invention, the selected therapy is further optimized by inclusion of delivery of steered pulses. In this embodiment, and referring to the terminology of the '519 patent, the following parameters are controlled to best direct the stimuli to the target sites: S₀-S₁ intervals; S₁-S₂ intervals; burst length; pulse amplitude; pulse width, or duration; scanning overdrive/underdrive; and the pulse, width and phase parameters of component pulses of composite cardioversion and defibrillation pulses. The implementation of this embodiment utilizes a set of three or more steering electrodes, as per the examples of Figures 1 and 7A.

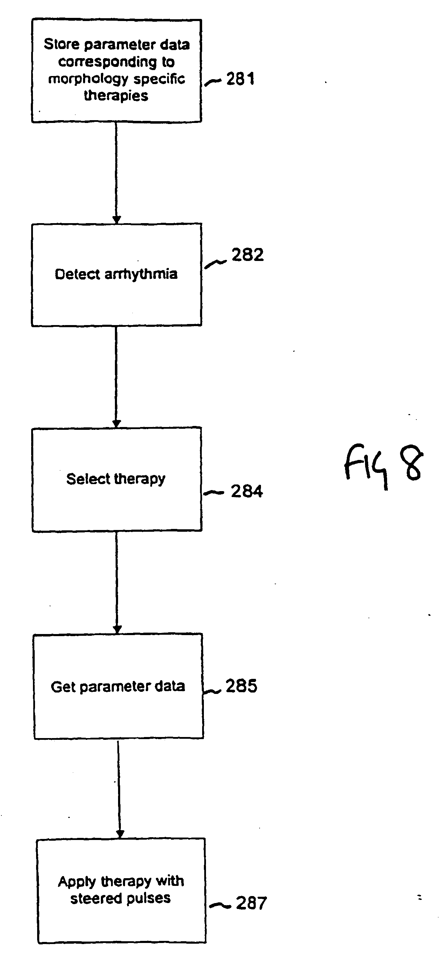

The flow diagram of Figure 8 shows the primary steps of a method of site specific therapy, utilizing the steering techniques of this invention. At block 281, the parameter data corresponding to different morphology specific therapies is stored in the stimulator device. At 282, the arrhythmia is detected, and at 284 the appropriate one of the available therapies is selected corresponding to the detected arrhythmia. Then, at 285 the parameter data for the selected therapy is obtained, and the therapy is applied at 287, using steered pulses generated with the selected parameter data.

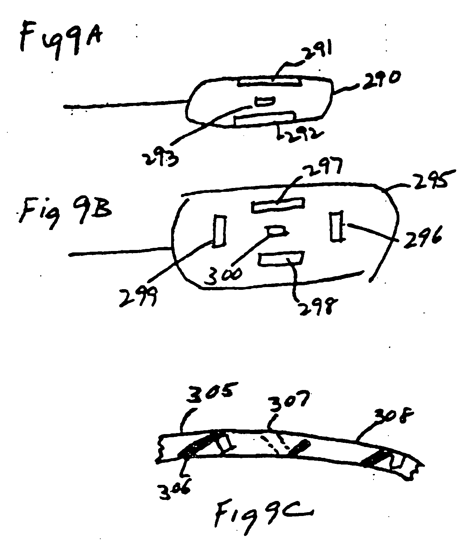

Figures 9A, 9B and 9C illustrates several alternative electrode configurations of the present invention. Those examples are illustrative of the fact that a number of different configurations beyond the three-ring electrode configuration can be used, and the invention is not limited in terms of the chosen configuration. As stated above, the distance between active electrodes for pacing applications is preferably greater than 2 cm, to provide for effective steering. In Figure 9A there is illustrated a distal portion of a lead having two longer electrodes 291,292, as well as a smaller electrode 293; 293 is suitably a common cathode, and 291 and 292 are respective anodes, with the pulse components delivered across pairs 291,292 and 293,292. The relatively longer anodes can provide better pulse direction control. This arrangement is more suitable for epicardial or patch-type electrodes, but may also be adapted to transvenous leads. Figure 9B illustrates a modified electrode configuration wherein four active electrodes 296, 297, 298, 299 are used in combination with a common electrode 300, to provide a selection of electrode pairs. In Figure 9C, there is presented a modification of a conventional transvenous lead, wherein three or more split ring electrodes are used, to provide additional flexibility in finding a best combination of pulse components for steering. In this embodiment, electrodes 306, 307 and 308 are shown on the distal end portion of a lead 305. Each electrode is illustrated as forming a portion of a ring, i.e., having a circumference less than 360 degrees, and being spaced axially along the lead length. Alternately, the two active electrodes 306,308 can have multiple turns, while common electrode 307 between each electrode may likewise have a circumference exceeding 360 degrees. For example, the two common electrodes may have multiple turns, while the common electrode between them has only one or a fraction of a turn. By this design, increased flexibility in finding suitable optimal parameters is provided.

There has been provided a system for steering of stimulus or shock pulses to cardiac sites. Effective electrode placement, sufficient interelectrode distance between active electrodes and appropriate control of the parameters of each pulse component the plural component composite pulse, all enable optimized steering for cardiac pacing, defibrillation or cardioversion.

## Claims

1. A pacing system comprising:
generator means (1, 2) for generating a composite pulse having at least two pulse components;
a lead (30) comprising first and second active electrodes and a common electrode for delivering said two pulse components at an area in or proximate to the patient's heart; and
control means (90) for controlling at least one parameter of each of said pulse components for steering said composite pulse to said selected site; wherein: said generator means comprises pulse generator means for generating pacing pulses for delivery to the patient's heart, having first pulse means for generating first pacing pulses between said first active electrode and said common electrode to form said composite pulse, said first pulses having controllable respective first magnitude, polarity and phase values, and second pulse means for generating second pacing pulses between said second active electrode and said common electrode substantially concurrently with said first pulses to form said composite pulse, said second pulses having controllable respective second magnitude, polarity and phase values; wherein said first pacing pulse is a first of said at least two pulse components and wherein said second pacing pulse is a second of said at least two pulse components; said composite pulse being a combination of a first pacing pulse and a second pacing pulse; and wherein said control means further comprises steering means which adjusts the relative magnitude and/or phase values of said first pulse means and said second pulse means so that said composite pulse is steered to an appropriate site.

2. The pacing system as described in claim 1 wherein said first and second active electrodes are anodes and wherein said common electrode is a cathode.

3. The pacing system as described in claim 1 further comprising capture detection means for determining when a pair of pulse outputs delivered from said lead electrodes has captured the patient's atrium.

4. The pacing system as described in any preceding claim, and further comprising switching means for switching predetermined ones of said generator outputs to selected ones of said electrodes.

5. The pacing system as described in any preceding claim, wherein said lead has a first set of electrodes (41, 42, 43) for delivering steering pulses to a patient's atrium and a second set of electrodes (31, 32, 33) for delivering steering pulses to a patient's ventricle.

6. The pacing system as described in any preceding claim, comprising sensing means for sensing patient natural cardiac signals at selected ones of said electrodes and further comprising sense switching means for switching respective ones of said electrodes to said sense switching means at controlled times.

7. A pacing system as claimed in claim 1 for dual chamber pacing of a patient; wherein
said lead is a pacing lead connected to said pulse generator means to receive said first and second pulses, said lead having a distal end portion adapted for positioning proximate to or in the patient's heart, said distal end portion having first and second active electrodes and a common electrode, and connecting means for connecting said first pacing pulses between said first electrode and said common electrode and for connecting said second pacing pulses between said second electrode and said common electrode; and
wherein said control means controls said pulse generator means to generate respective steered ventricular and atrial pacing pulses, each of which steered pulses is a combination of a said first and a second pulse, and wherein said steering means further controls said first and second pulse means so as to select a first set of values of magnitude, polarity and phase for each of said first and second pulses when said pulse generator generates a ventricular pacing pulse, and a second set of values of magnitude, polarity and phase for each of said first and second pulses when said pulse generator generates an atrial pacing pulse.

8. The system as described in any preceding claim, wherein said control means comprises timing means for controlling the timing of respective generated ventricular and atrial pulses.

9. The system as described in claim 8, comprising sensing means connected to said active electrodes and said common electrode for sensing electrical signals from the patient's heart, and wherein said timing means controls said timing as a function of said sensed electric signals.

10. The system as described in claim 9, comprising additional sensing means located apart from said lead distal end portion for sensing patient cardiac signals, and determining means for determining from said sensed cardiac signals whether they represent atrial or ventricular signals.

11. The system as described in claim 3, further comprising
threshold detecting means operative together with said capture detection means for determining capture threshold for said steered atrial pulses.

12. The system as described in claim 11, further comprising search means for varying at least one parameters of said first and second pacing pulses of said atrial pulse, and searching for a set of parameter values corresponding to optimum threshold for pacing the patient's atrium.

13. The system as described in any preceding claim wherein said control means comprises parameter adjustment means for adjusting one or more of the amplitude, pulse width and phase of each said pulse component.

14. The system as described in claim 13, wherein said parameter adjustment means comprises amplitude increment means for incrementing the amplitude of a said pulse component by a predetermined increment.

15. The system as described in claim 13 or 14, wherein said parameter adjustment means comprises pulse width means for incrementing the pulse width of a said pulse component by a predetermined increment.

16. The system as described in claim 13, 14 or 15, wherein said parameter adjustment means comprises phase means for adjusting the phase of a said pulse component by a predetermined increment.

17. The system as described in any preceding claim, wherein said generator means further comprises defibrillator means for generating a composite defibrillation pulse.

18. The system as described in any preceding claim, wherein each of said electrodes is separated by a distance of at least 1 cm.

19. The system as described in any of claims 1 to 16, wherein said two active electrodes are separated by a distance of at least 2 cm.

## Patentansprüche

1. Stimulationssystem mit:
Generatormitteln (1, 2) zum Generieren eines zusammengesetzten Pulses mit wenigstens zwei Pulskomponenten;
einer Leitung (30) mit ersten und zweiten aktiven Elektroden und einer gemeinsamen Elektrode zum Abgeben der zwei Pulskomponenten an eine Region im Herzen oder in der Nähe des Herzens eines Patienten; und
Steuermitteln (90) zum Steuern wenigstens eines Parameters einer jeden der Pulskomponenten zum Lenken des zusammengesetzten Pulses zu dem ausgewählten Ort; wobei:
die Generatormittel Pulsgeneratormittel zum Generieren von Stimulationspulsen zur Abgabe an das Herz des Patienten aufweisen, mit ersten Pulsmitteln zum Generieren erster Stimulationspulse zwischen der ersten aktiven Elektrode und der gemeinsamen Elektrode zur Bildung des zusammengesetzten Pulses, wobei die ersten Pulse steuerbare zugehörige erste Größen-, Polaritäts- und Phasenwerte aufweisen, und zweiten Pulsmitteln zum Erzeugen zweiter Stimulationspulse zwischen der zweiten aktiven Elektrode und der gemeinsamen Elektrode im wesentlichen gleichzeitig mit den ersten Pulsen zum Bilden des zusammengesetzten Pulses, wobei die zweiten Pulse steuerbare zugehörige zweite Magnituden-, Polaritäts- und Phasenwerte aufweisen; wobei der erste Stimulationspuls ein erster der wenigstens zwei Pulskomponenten und wobei der zweite Stimulationspuls ein zweiter der wenigstens zwei Pulskomponenten ist; wobei der zusammengesetzte Puls eine Kombination eines ersten Stimulationspulses und eines zweiten Stimulationspulses ist; und wobei die Steuermittel ferner Lenkmittel aufweisen, die die relativen Magnituden- und/oder Phasenwerte der ersten Pulsmittel und der zweiten Pulsmittel einstellen, so dass der zusammengesetzte Puls an einen geeigneten Ort gelenkt wird.

2. Stimulationssystem nach Anspruch 1, bei dem die ersten und zweiten aktiven Elektroden Anoden sind, und bei dem die gemeinsame Elektrode eine Kathode ist.

3. Stimulationssystem nach Anspruch 1, das ferner Beaufschlagungsbestimmungsmittel aufweist, um zu bestimmen, wann ein Paar von den Leitungselektroden abgegebener Pulse das Atrium des Patienten beaufschlagt.

4. Stimulationssystem nach einem der vorstehenden Ansprüche, und ferner mit Schaltmitteln zum Schalten bzw. Anlegen von vorbestimmten Generatorausgaben der Generatorausgaben an ausgewählte Elektroden der Elektroden.

5. Stimulationssystem nach einem der vorstehenden Ansprüche, bei dem die Leitung einen ersten Satz Elektroden (41, 42, 43) zur Abgabe von Lenkpulsen an das Atrium eines Patienten und einen zweiten Satz Elektroden (31, 32, 33) zur Abgabe von Lenkpulsen an das Ventrikel eines Patienten aufweist.

6. Stimulationssystem nach einem der vorstehenden Ansprüche, das Erfassungsmittel zum Erfassen von natürlichen Herzsignalen des Patienten bei bzw. mit ausgewählten Elektroden der Elektroden aufweist und ferner ausgewählten Elektroden der Elektroden aufweist und ferner Erfassungsschaltmittel zum Schalten zugehöriger Elektroden der Elektroden zu bzw. an die Erfassungsschaltmittel zu gesteuerten Zeiten aufweist.

7. Stimulationssystem nach Anspruch 1 zur Zweikammerstimulation eines Patienten; wobei
die Leitung eine Stimulationsleitung ist, die mit den Pulsgeneratormitteln zum Empfangen der ersten und zweiten Pulse verbunden ist, wobei die Leitung einen distalen Endabschnitt aufweist, der dafür eingerichtet ist, proximal zu oder in dem Herzen des Patienten positioniert zu werden, wobei der distale Endabschnitt erste und zweite aktive Elektroden und eine gemeinsame Elektrode und Verbindungsmittel zum Verbinden der ersten Stimulationspulse zwischen der ersten Elektrode und der gemeinsamen Elektrode und zum Verbinden der zweiten Stimulationspulse zwischen der zweiten Elektrode und der gemeinsamen Elektrode aufweist; und
wobei die Steuermittel die Pulsgeneratormittel zur Erzeugung jeweiliger gelenkter ventrikulärer und atrialer Stimulationspulse steuern, wobei jeder der gelenkten Pulse eine Kombination eines ersten und eines zweiten Pulses ist, und wobei die Lenkmittel ferner die ersten und zweiten Pulsmittel steuern, um einen ersten Satz von Werten der Magnitude, der Polarität und der Phase für jeden der ersten und zweiten Pulse auszuwählen, wenn der Pulsgenerator einen ventrikulären Stimulationspuls erzeugt, und einen zweiten Satz von Werten der Magnitude, der Polarität und der Phase für jeden der ersten und zweiten Pulse, wenn der Pulsgenerator einen atrialen Stimulationspuls erzeugt.

8. System nach einem der vorstehenden Ansprüche, bei dem die Steuermittel Timingmittel bzw. Zeitsteuerungsmittel zum Steuern des Timings der zugehörigen erzeugten ventrikulären und atrialen Pulse aufweisen.

9. System nach Anspruch 8, das Erfassungsmittel aufweist, die mit den aktiven Elektroden und mit der gemeinsamen Elektrode zur Erfassung von elektrischen Signalen von dem Herz des Patienten verbunden sind, und bei dem die Timingmittel das Timing als eine Funktion der erfassten elektrischen Signale steuern.

10. System nach Anspruch 9, das zusätzlich Erfassungsmittel, die entfernt bzw. gesondert von dem distalen Endabschnitt der Leitung zur Erfassung von Herzsignalen des Patienten angeordnet sind, und Bestimmungsmittel, um aus den erfassten Herzsignalen zu bestimmen, ob sie atriale oder ventrikuläre Signale repräsentieren, aufweist.

11. System nach Anspruch 3, das ferner Schwellwertbestimmungsmittel aufweist, die zusammen mit den Erfassungsbestimmungsmitteln zum Bestimmen des Erfassungsschwellwertes für die gelenkten atrialen Pulse operativ bzw. aktiv sind.

12. System nach Anspruch 11, das ferner Suchmittel zum Variieren wenigstens eines Parameters der ersten und zweiten Stimulationspulse des atrialen Pulses und zum Suchen eines Satzes von Parameterwerten, die einem optimalen Schwellwert zur Stimulation des Atriums des Patienten entsprechen, aufweist.

13. System nach einem der vorstehenden Ansprüche, bei dem die Steuermittel Parametereinstellmittel zum Einstellen der Amplitude, der Pulsweite und/oder der Phase einer jeden Pulskomponente aufweisen.

14. System nach Anspruch 13, bei dem die Parametereinstellmittel Amplitudeninkrementmittel zum Inkrementieren der Amplitude einer der Pulskomponenten um ein vorbestimmtes Inkrement aufweisen.

15. System nach Anspruch 13 oder 14, bei dem die Parametereinstellmittel Pulsweitenmittel zum Inkrementieren der Pulsweite einer der Pulskomponenten um ein vorbestimmtes Inkrement aufweisen.

16. System nach Anspruch 13, 14 oder 15, bei dem die Parametereinstellmittel Phasenmittel zum Einstellen der Phase einer der Pulskomponenten um ein vorbestimmtes Inkrement aufweisen.

17. System nach einem der vorstehenden Ansprüche, bei dem die Generatormittel ferner Defibrillatormittel zum Erzeugen eines zusammengesetzten Defibrillationspulses aufweisen.

18. System nach einem der vorstehenden Ansprüche, bei dem alle Elektroden durch einen Abstand von wenigstens 1 cm voneinander getrennt sind.

19. System nach einem der Ansprüche 1 bis 16, bei dem zwei aktive Elektroden durch einen Abstand von wenigstens 2 cm voneinander getrennt sind.

## Revendications

1. Système de stimulation comportant:
des moyens de génération (1, 2) pour générer une impulsion composite ayant au moins deux composantes d'impulsion ;
une dérivation (30) comportant des première et seconde électrodes actives et une électrode commune pour délivrer lesdites deux composantes d'impulsion à une zone dans le coeur du patient ou à proximité de celui-ci ; et
des moyens de commande (90) pour commander au moins un paramètre de chacune desdites composantes d'impulsion pour piloter ladite impulsion composite vers ledit site sélectionné ; dans lequel :
lesdits moyens de génération comportent des moyens de génération d'impulsions pour générer des impulsions de stimulation en vue d'un envoi vers le coeur du patient, ayant des premiers moyens d'impulsions pour générer des premières impulsions de stimulation entre ladite première électrode active et ladite électrode commune afin de former ladite impulsion composite, lesdites premières impulsions ayant des premières valeurs d'amplitude, de polarité et de phase respectives pouvant être commandées, et des seconds moyens d'impulsions pour générer des secondes impulsions de stimulation entre ladite seconde électrode active et ladite électrode commune substantiellement simultanément avec lesdites premières impulsions pour former ladite impulsion composite, lesdites secondes impulsions ayant des secondes valeurs d'amplitude, de polarité et de phase respectives pouvant être commandées ; dans lequel ladite première impulsion de stimulation est une première desdites au moins deux composantes d'impulsion et dans lequel ladite seconde impulsion de stimulation est une seconde desdites au moins deux composantes d'impulsion ; ladite impulsion composite étant une combinaison d'une première impulsion de stimulation et d'une seconde impulsion de stimulation ; et dans lequel lesdits moyens de commande comportent en outre des moyens de pilotage qui ajustent les valeurs d'amplitude et/ou de phase relatives desdits premiers moyens d'impulsion et desdits seconds moyens d'impulsion de sorte que ladite impulsion composite est pilotée vers un site appropriée.

2. Système de stimulation comme décrit dans la revendication 1 dans lequel lesdites première et seconde électrodes actives sont des anodes et dans lequel ladite électrode commune est une cathode.

3. Système de stimulation comme décrit dans la revendication 1 comportant en outre des moyens de détection de capture pour déterminer lorsqu'une paire de sorties d'impulsions délivrée par lesdites électrodes de dérivation a capturé l'atrium du patient.

4. Système de stimulation comme décrit dans l'une quelconque des revendications précédentes, et comportant en outre des moyens de commutation pour commuter des sorties prédéterminées parmi lesdits sorties du générateur sur des électrodes sélectionnées parmi lesdites électrodes.

5. Système de stimulation comme décrit dans l'une quelconque des revendications précédentes, dans lequel ladite dérivation a un premier ensemble d'électrodes (41, 42, 43) pour délivrer des impulsions de pilotage à l'atrium d'un patient et un second ensemble d'électrodes (31, 32, 33) pour délivrer des impulsions de pilotage à un ventricule d'un patient.

6. Système de stimulation comme décrit dans l'une quelconque des revendications précédentes, comportant des moyens de détection pour détecter des signaux cardiaques naturels du patient au niveau d'électrodes sélectionnées parmi lesdites électrodes et comportant en outre des moyens de commutation de détection pour commuter des électrodes respectives parmi lesdites électrodes sur lesdits moyens de commutation de détection à des instants commandés.

7. Système de stimulation selon la revendication 1 pour une stimulation double chambre d'un patient ; dans lequel ladite dérivation est une dérivation de stimulation connectée auxdits moyens de génération d'impulsions pour recevoir des première et seconde impulsions, ladite dérivation ayant une partie d'extrémité distale adaptée pour être positionnée dans le coeur du patient ou à proximité de celui-ci, ladite partie d'extrémité distale ayant des première et seconde électrodes actives et une électrode commune, et des moyens de connexion pour connecter lesdites premières impulsions de stimulation entre ladite première électrode et ladite électrode commune et pour connecter lesdites secondes impulsions de stimulation entre ladite seconde électrode et ladite électrode commune ; et
dans lequel lesdits moyens de commande commandent lesdits moyens de génération d'impulsions pour générer des impulsions de stimulation ventriculaire et auriculaire pilotées respectives, chacune de ces impulsions pilotées est une combinaison de ladite première et de ladite seconde impulsion, et dans lequel lesdits moyens de pilotage commandent en outre lesdits premiers et seconds moyens d'impulsions de manière à sélectionner un premier ensemble de valeurs d'amplitude, de polarité et de phase pour chacune desdites première et seconde impulsions lorsque ledit générateur d'impulsions génère une impulsion de stimulation ventriculaire, et un second ensemble de valeurs d'amplitude, de polarité et de phase pour chacune desdites première et seconde impulsions lorsque ledit générateur d'impulsions génère une impulsion de stimulation auriculaire.

8. Système comme décrit dans l'une quelconque des revendications précédentes, dans lequel lesdits moyens de commande comportent des moyens de synchronisation ou des moyens de timing pour commander la synchronisation d'impulsions ventriculaires et auriculaires respectives.

9. Système comme décrit dans la revendication 8, comportant des moyens de détection connectés auxdites électrodes actives et à ladite électrode commune pour détecter des signaux électriques provenant du coeur du patient, et dans lequel lesdits moyens de synchronisation commandent ladite synchronisation en fonction desdits signaux électriques détectés.

10. Système comme décrit dans la revendication 9, comportant des moyens de détection supplémentaires situés à l'extérieur de ladite partie d'extrémité distale pour détecter des signaux cardiaques du patient, et des moyens de détermination pour déterminer à partir desdits signaux cardiaques détectés si ceux-ci représentent des signaux auriculaires ou ventriculaires.

11. Système comme décrit dans la revendication 3, comportant en outre
des moyens de détection de seuil opérationnels conjointement avec lesdits moyens de détection de capture pour déterminer un seuil de capture pour lesdites impulsions auriculaires pilotées.

12. Système comme décrit dans la revendication 11, comportant en outre des moyens de recherche pour faire varier au moins un paramètre desdites première et seconde impulsions de stimulation de ladite impulsion auriculaire, et rechercher un ensemble de valeurs de paramètre correspondant à un seuil optimum pour stimuler l'atrium d'un patient.

13. Système comme décrit dans l'une quelconque des revendications précédentes dans lequel lesdits moyens de commande comportent des moyens d'ajustement de paramètre pour ajuster un ou plusieurs paramètres parmi l'amplitude, la largeur d'impulsion et la phase de chacune desdites composantes d'impulsion.

14. Système comme décrit dans la revendication 13, dans lequel lesdits moyens d'ajustement de paramètre comportent des moyens d'incrément d'amplitude pour incrémenter l'amplitude de ladite composante d'impulsion d'un incrément prédéterminé.

15. Système comme décrit dans la revendication 13 ou 14, dans lequel lesdits moyens d'ajustement de paramètre comportent des moyens de largeur d'impulsion pour incrémenter la largeur d'impulsion de ladite composante d'impulsion d'un incrément prédéterminé.

16. Système comme décrit dans la revendication 13, 14 ou 15, dans lequel lesdits moyens d'ajustement de paramètre comportent des moyens de phase pour ajuster la phase de ladite composante d'impulsion d'un incrément prédéterminé.

17. Système comme décrit dans l'une quelconque des revendications précédentes, dans lequel lesdits moyens de génération comportent en outre des moyens de défibrillation pour générer une impulsion de défibrillation composite.

18. Système comme décrit dans l'une quelconque des revendications précédentes, dans lequel chacune desdites électrodes est séparée d'une distance d'au moins 1 cm.

19. Système comme décrit dans l'une quelconque des revendications 1 à 16, dans lequel lesdites deux électrodes actives sont séparées d'un distance d'au moins 2 cm.
